(19) Europäisches Patentamt

European Patent Office

Office européen des brevets

(11) **EP 4 629 632 A1**

(12) **EUROPEAN PATENT APPLICATION**

(43) Date of publication:
08.10.2025 Bulletin 2025/41

(21) Application number: 24168719.3

(22) Date of filing: 05.04.2024

(51) International Patent Classification (IPC):
*H04N 19/593* $^{(2014.01)}$ *H04N 19/91* $^{(2014.01)}$
*G03B 42/02* $^{(2021.01)}$ *A61B 6/00* $^{(2024.01)}$

(52) Cooperative Patent Classification (CPC):
**H04N 19/593; A61B 6/5205; A61B 6/56;**
**G03B 42/02; H04N 19/91**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
- **Rehfeld, Niklas**
**90408 Nürnberg (DE)**
- **Hofmann, Günter**
**96047 Bamberg (DE)**
- **Hofmann, Philipp**
**91320 Ebermannstadt (DE)**

(74) Representative: **Siemens Healthineers**
**Patent Attorneys**
**Postfach 22 16 34**
**80506 München (DE)**

(54) **COMPRESSING DATA OF A COMPUTED TOMOGRAPHY SYSTEM**

(57) For compressing data of a computed tomography, CT, system (1), detector data (5) is received from an X-ray detector of the CT system (1), pre-compressed data (6) is generated by compressing the detector data (5) using a lossy compression method, and fully compressed data (7) is generated depending on the pre-compressed data (6) using a lossless compression method.

EP 4 629 632 A1

## Description

The present invention is directed to a computer-implemented method for compressing data of a computed tomography, CT, system, wherein detector data is received from an X-ray detector of the CT system. The invention is further directed to a corresponding computer-implemented method for compressing and decompressing data of a CT system and to a computer-implemented method for generating a CT reconstruction, wherein such a computer-implemented method for compressing and decompressing data of a CT system is carried out. The invention is also directed to a data processing arrangement for carrying out such methods and to a corresponding computer program product as well as to a CT system.

In modern computed tomography, CT, massive amounts of potentially multi-dimensional data are generated by the X-ray detector, which must be transmitted via a bandwidth-limited connection, in particular from a rotating part of the CT scanner carrying the X-ray detector to a stationary part of the CT scanner and further to a computer for CT reconstruction, for example. The multiple dimensions of the data may concern, for example, detector pixel coordinates values, a detector index in case of dual source scanners, et cetera. Especially with spectral CT systems based on photon counting technology, enormous amounts of multi-dimensional data need to be transferred, processed, and stored. Additional dimensions of the data can therefore concern the different energy bin information and other data, such as coincidence information. The potential data rate to be transmitted can be in the range of hundreds of GBit/s.

Lossy data compression may be used to limit the required bandwidth and/or storage space. However, only a limited loss of information is acceptable, which limits the achievable compression ratio. Lossless compression, on the other hand, is potentially slow and might not yield sufficient compression ratios either.

In document US 9,078,625 B2, a method for transmitting measurement data from a transmitter system to a receiver system by way of a transmission link of a medical device is described. In the measurement data is transformed to output values and, after transmission, back-transformed again, the values of the input data lying between a maximum value and a minimum value. An assignment function is used for compression to allocate an output value to every value of the input data, wherein a root function is used as the assignment function for at least some of the values. For example, the assignment function may be defined piecewise as a root function and piecewise as a linear function.

It is an objective of the present invention to increase the achievable compression ratio and/or processing speed and at the same time limit a loss of information, when compressing data of a CT system.

This objective is achieved by the respective subject matter of the independent claims. Further implementations and preferred embodiments are subject matter of the dependent claims.

The invention is based on the idea to implement a two-stage data compression scheme, which pre-compresses the detector data using lossy compression and then further compresses the pre-compressed data using lossless compression.

According to an aspect of the invention, a computer-implemented method for compressing data of a CT system is provided. Detector data is received from an X-ray detector of the CT system. Pre-compressed data is generated by compressing the detector data using a lossy compression method. Fully compressed data is generated depending on the pre-compressed data using a lossless compression method.

Unless stated otherwise, all steps of the computer-implemented method may be performed by a first data processing system, which comprises at least one first data processing device. In particular, the at least one first data processing device is configured or adapted to perform the steps of the computer-implemented method. For this purpose, the at least one first data processing device may for example store a first computer program comprising first instructions which, when executed by the at least one first data processing device, cause the at least one first data processing device to execute the computer-implemented method. The expressions "data processing system" and "at least one data processing device" may be used interchangeably, here and in the following. This holds also for respective expressions derived therefrom.

In case the at least one first data processing device comprises two or more first data processing devices, certain steps carried out by the at least one first data processing device may also be understood such that different first data processing devices carry out different steps or different parts of a step. In particular, it is not required that each first data processing device carries out the steps completely. In other words, carrying out the steps may be distributed amongst the two or more first data processing devices.

Receiving the detector data may comprise, for example, capturing and/or reading a computer-readable data memory and/or receiving from a data storage unit, for example a database, and/or receiving a data stream with the detector data, for example from the X-ray detector. The detector data may, in particular, be raw data generated by the X-ray detector. Generating the detector data is not part of the computer-implemented method. However, from each implementation of the computer-implemented method, a respective implementation of a method for compressing data of a CT system, which is not purely computer-implemented, is obtained by including respective steps of generating the detector data by the X-ray detector.

The X-ray detector comprises, in particular, a detector pixel array corresponding to a two-dimensional array of detector pixels, wherein the detector pixels are not necessarily arranged in a plane, but can also be arranged on a curved surface, for example part of an inner surface of a spherical surface or a cylindrical surface. The detector pixels may also be arranged in pixel groups, so that the detector pixel array contains an array of pixel groups, each pixel group in turn containing a sub-array of individual detector pixels. The individual detector pixels of a pixel group can, for example, be directly adjacent to each other, whereas distances can be provided between different pixel groups. However, other architectures of the detector pixel array are also possible. It is also possible for the detector pixel array to have several different hardware components that are also arranged in the form of an array. Each hardware component then contains a plurality of detector pixels, which may be grouped into pixel groups within the hardware component. Further hierarchical gradations are also possible.

The detector data comprises, in particular, one or more respective pixel values for each of a plurality of detector pixels of the X-ray detector, in particular of the detector pixel array. The detector data may comprise one or more respective pixel values for all detector pixels of the detector pixel array or only for a fraction of them, for example for all detector pixels within a predefined contiguous spatial area, which corresponds, for example, to a spatial structure of the detector pixel array as described above. The one or more pixel values may be given as individual, scalar values or as entries of one or more vectors, one or more matrices or one or more higher-dimensional tensors, especially in case the CT system is a spectral CT system with a photon counting X-ray detector.

The one or more pixel values, the pre-compressed data and the fully compressed data are, for example, integer values. The pre-compressed data are denoted as such, since they are the result of the lossy compression method but are yet further compressed by the lossless compression method.

By means of the computer-implemented method according to the invention, a two-stage data compression scheme is implemented, which pre-compresses the detector data using the lossy compression method and then further compresses the pre-compressed data using the lossless compression method. Consequently, the compression ratio achieved by the lossy compression method may be adjusted or limited to avoid a significant loss of information. Furthermore, details of the lossy compression method may partly be physically motivated, taking in account, for example, the noise level of the detector data, the Poisson distribution of the X-ray photons, et cetera, which increases the efficiency of the lossy compression method. The limited compression ratio may then be compensated by the subsequent lossless compression method. Known lossy and lossless compression methods may be used. Preferred lossy and lossless compression methods will be described further below.

According to several implementations, the detector data corresponds to a frame of a frame sequence, which comprises the frame and at least one further frame. For each of the at least one further frame, respective further detector data is received, for example from the X-ray detector, and respective further pre-compressed data is generated by compressing the respective further detector data using the lossy compression method. The fully compressed data is generated by compressing joint pre-compressed data, which comprises or consists of the pre-compressed data for the frame and the further pre-compressed data for the at least one further frame.

Therein, that the detector data corresponds to the frame can be understood such that it is or has been generated by the X-ray detector during the frame. In other words, while individual frames are pre-compressed separately by means of the lossy compression method, the pre-compressed data of two or more frames, in particular, of a batch of frames, is compressed jointly by the lossless compression method. In this way, the overall efficiency and/or speed of the method is improved.

According to several implementations, the lossless compression method is an entropy coding method, for example, the lossless compression method is an asymmetric numeral systems, ANS, method.

Such implementations are particularly beneficial, since the lossy compression method may reduce the entropy of the data to be compressed by the entropy coding method. Thus, the compression ratio of the lossless compression method and, consequently, the overall compression ratio are increased.

According to several implementations, the detector data comprises a respective pixel value for each of a plurality of detector pixels of the X-ray detector. The lossy compression method comprises a pixel-wise lossy compression, wherein a respective pre-compressed pixel value is computed for each detector pixel of the plurality of detector pixels, in particular depending on the respective pixel value.

The pixel-wise lossy compression implies that there is a one-to-one correspondence between a pixel value of a given detector pixel and the respective pre-compressed pixel value.

For example, the pixel-wise lossy compression comprises, for each detector pixel of the plurality of detector pixels, computing the pre-compressed pixel value for the respective detector pixel using a predefined lossy compression function, whose argument depends on, for example comprises or consists of, the respective pixel value. The lossy compression function is also denoted as compression function in the following.

The compression function may also be denoted as assignment function. The compression function may for example be an assignment function as described in document US 9,078,625 B2 mentioned above.

For example, a codomain of the compression function may be a set of integers, in particular given by $[N_0, N] \cap \mathbb{N}$, wherein $N_0$ is a predefined positive integer or zero and $N$ is a predefined positive integer, which is greater than $N_0$. In particular, die cardinality of the codomain is smaller than a cardinality of a domain of the compression function, which corresponds to a set of integers as well.

For example, the cardinality of the codomain is given by a predefined bit depth denoted by m. In particular, the cardinality of the codomain may be $2^m$. For example, the codomain may be given by $\{0, 1, 2, ..., 2^m - 1\}$. The bit depth m may for example be an integer from 5 to 15, for example 8 to 15.

According to several implementations, the pixel-wise lossy compression comprises, for each detector pixel of the plurality of detector pixels, receiving historic pixel values of each environment pixel within a predefined historic environment of the respective detector pixel. A predictor value is computed for each detector pixel of the plurality of detector pixels depending on the historic pixel values of the environment pixels, of the respective detector pixel, in particular all environment pixels of the respective detector pixel, and independent of the pixel value of the respective detector pixel. The pre-compressed data, in particular a respective part of the pre-compressed data, is generated depending on the pre-compressed pixel value for the respective detector pixel and the predictor value for the respective detector pixel.

In particular, the predictor value may be used to estimate the pre-compressed pixel value for the respective detector pixel based on the historic pixel values. Thus, the efficiency of the pixel-wise lossy compression is improved.

The historic environment is predefined for each detector pixel of the plurality of detector pixels in a unique way, in particular in terms of one or more rules. Therein, all environment pixels of the historic environment have undergone the pixel-wise lossy compression before the pixel-wise lossy compression is applied to the respective pixel under consideration. In this sense the historic environment may be considered "historic". In particular, the historic environment does not comprise the respective pixel under consideration itself. Consequently, the respective pre-compressed pixel values of the historic environment are readily available during the decompression process. The historic environment may comprise environment pixels being pixels from a previous frame. Alternatively or in addition, the historic environment may comprise environment pixels being pixels from the same frame as the respective pixel under consideration. Consequently, the one or more rules defining the historic environment may include an initialization rule for an initial pixel. In this case, the historic environment may be empty, for example, and the respective historic pixel values may be set to zero or to predefined other values for this case.

For a given detector pixel at a given frame, the environment pixels may comprise neighboring pixels of the given detector pixel. For example, the historic environment may consist of a contiguous area of detector pixels excluding the given detector pixel. In other words, the historic environment is a predefined neighborhood of the given detector pixel. This is, however, not necessarily the case. In particular, the historic environment may also comprise environment pixels, whose pixel values are expected to be correlated with the pixel value of the given detector pixel. This may for example be the case for pixels in similar parts of the detector array or pixels within the same sub-array of the detector array, et cetera. The number of the environment pixels within the historic environment may be equal to one or may be greater than one. In some implementations, the historic environment of a given pixel may even consist of the same pixel in the previous frame.

In some implementations, the argument of the compression function does not only depend on the respective pixel value but also on the historic pixel values of the environment pixels of the respective detector pixel. For example, the argument of the compression function may comprise or consist of the respective pixel value and the historic pixel values of the environment pixels of the respective detector pixel. In this way, the correlation between different pixels of the detector array may be further exploited to improve the efficiency of the lossy compression method.

According to several implementations, the predictor value is computed as a weighted sum or weighted average of the historic pixel values of the environment pixels of the respective detector pixel.

Consequently, a particularly simple way of generating the predictor value with very limited computational costs is achieved.

According to several implementations, the predictor value is computed by applying a trained machine learning model, MLM, to input data comprising or consisting of the historic pixel values of the environment pixels of the respective detector pixel.

In general terms, a trained MLM may mimic cognitive functions that humans associate with other human minds. In particular, by training based on training data the MLM may be able to adapt to new circumstances and to detect and extrapolate patterns. Another term for a trained MLM is "trained function".

In general, parameters of an MLM can be adapted or updated by means of training. In particular, supervised training, semi-supervised training, unsupervised training, reinforcement learning and/or active learning can be used. Furthermore, representation learning, also denoted as feature learning, can be used. In particular, the parameters of the MLMs can be adapted iteratively by several steps of training. In particular, within the training a certain loss function, also denoted as cost function, can be minimized. In particular, within the training of an artificial neural network, ANN, the backpropagation algorithm can be used.

In particular, an MLM can comprise an ANN, a support vector machine, a decision tree and/or a Bayesian network, and/or the MLM can be based on k-means clustering, Q-learning, genetic algorithms and/or association rules. In particular, an ANN can be or comprise a deep neural network, a convolutional neural network or a convolutional deep neural network. Furthermore, an ANN can be an adversarial network, a deep adversarial network and/or a generative adversarial network.

According to several implementations, a pre-compressed predictor value is computed based on the predictor value for the respective detector pixel. A difference value between the pre-compressed pixel value for the respective detector pixel and the pre-compressed predictor value for the respective detector pixel is computed. The pre-compressed data, in particular the respective part of the pre-compressed data, is generated depending on the difference values, in particular offset difference values, computed for the plurality of detector pixels.

The pre-compressed predictor value is, in particular, computed based on the predictor value in the same way or using the same computation as for computing the pre-compressed pixel value based on the respective pixel value. In particular, the pre-compressed predictor value may be computed by applying the compression function to the predictor value or, in respective implementations, to the predictor value and the historic pixel values for the respective detector pixel.

Since the historic pixel values are potentially correlated to the pixel value of the respective detector pixel, the pre-compressed predictor value can be considered as a guess or prediction of the pre-compressed pixel value. Thus, the difference between them is potentially a small number. Thus the efficiency of the pixel-wise lossy compression is improved.

For example, the pre-compressed data may comprise, for each of the plurality of detector pixels, the value

$$(D(x) + 2^m) \, mod \, 2^m$$

within the codomain of the compression function. Therein, m is the predefined bit depth, x is the pixel value of the respective detector pixel, and $D(x) = L(x) - L(P_x)$ is the difference value between the pre-compressed pixel value for the respective detector pixel and the pre-compressed predictor value for the respective detector pixel. Therein, $L$ denotes the compression function and $P_x$ denotes predictor value for the respective detector pixel. Adding the term $2^m$ to the difference value ensures that the $(D(x) + 2^m)$ is positive and the $mod \, 2^m$ ensures that $(D(x) + 2^m) \, mod \, 2^m$ lies within the codomain of the compression function. The value $(D(x) + 2^m) \, mod \, 2^m$ may be denoted as offset difference value.

According to a further aspect of the invention, a computer-implemented method for compressing and decompressing data of a CT system is provided. Therein, a computer-implemented method for compressing the data of the CT system according to the invention is carried out by a first data processing system.

In a first alternative, the fully compressed data is transmitted from the first data processing system to a second data processing system, in particular via a transmission link of the CT system, for example a slip ring of the CT system, or the fully compressed data is stored to a storage device by the first data processing system and the fully compressed data is read out from the storage device by the second data processing system.

The pre-compressed data is recovered by the second data processing system based on the fully compressed data by reversing the lossless compression method, in particular the entropy coding method, for example the ANS method. Decompressed data is generated by the second data processing system based on the recovered pre-compressed data by reversing the lossy compression method.

In a second alternative, the fully compressed data is stored to the storage device by the first data processing system and the fully compressed data is read out from the storage device by the first data processing system at a later time. Then, the pre-compressed data is recovered by the first data processing system based on the fully compressed data by reversing the lossless compression method, in particular the entropy coding method, for example the ANS method. The decompressed data is generated by the first data processing system based on the recovered pre-compressed data by reversing the lossy compression method.

According to several implementations of the computer-implemented method for compressing and decompressing data of the CT system, reversing the lossy compression method comprises a pixel-wise lossy decompression, wherein a respective decompressed pixel value is computed for each detector pixel of the plurality of detector pixels.

The pixel-wise lossy decompression is, in particular, a reversal of the pixel-wise lossy compression and may involve, for example, applying an inverse of the compression function accordingly.

According to several implementations of the computer-implemented method for compressing and decompressing data of the CT system, the computer-implemented method for compressing the data of the CT system is carried out such that the pre-compressed data is generated depending on the pre-compressed pixel value for the respective detector pixel and the predictor value for the respective detector pixel, as described above.

Carrying out the pixel-wise lossy decompression comprises, for each detector pixel of the plurality of detector pixels, receiving recovered historic pixel values of each environment pixel within the historic environment of the respective detector pixel, re-computing the predictor value for the respective detector pixel depending on the recovered historic pixel values of the environment pixels of the respective detector pixel, and computing the respective decompressed pixel value depending on the recovered pre-compressed data for the respective detector pixel and the re-computed predictor value for the respective detector pixel.

In particular, a pixel order of the pixel-wise lossy decompression is identical to a pixel order of the pixel-wise lossy compression. Therefore, when the decompressed pixel value for a given detector pixel is to be computed, the decompressed pixel values for the environment pixels within the historic environment or, in other words, the historic pixel values, already have been computed before and are available. The predictor value can therefore be recomputed based on the recovered historic pixel values in the same way as the prediction value was computed during the compression phase based on the historic pixel values.

According to several implementations of the computer-implemented method for compressing and decompressing data of the CT system, the computer-implemented method for compressing the data of the CT system is carried out such that the pre-compressed data is generated depending on the difference values for the plurality of detector pixels, in particular the offset difference values, as described above.

A further pre-compressed predictor value is computed based on the re-computed predictor value, for example by applying the inverse of the compression function to the re-computed predictor value. For each detector pixel of the plurality of detector pixels, the difference value for the respective detector pixel, in particular the offset difference value mod $2^m$, is recovered based on the recovered pre-compressed data. A sum value of the recovered difference value for the respective detector pixel and the further pre-compressed predictor value for the respective detector pixel is computed or a sum value of the recovered offset difference value mod $2^m$ and the further pre-compressed predictor value. The respective decompressed pixel value is computed depending on the sum value. In particular, computing the sum value reverses computing the difference value during compression.

For example, the respective decompressed pixel value is computed applying an inverse of the compression function to an argument, which depends on, in particular comprises or consists of, the respective sum value.

According to a further aspect of the invention, a computer-implemented method for generating a CT reconstruction is provided. Therein, a computer-implemented method for compressing and decompressing data of a CT system according to the invention is carried out. The CT reconstruction is generated depending on the decompressed data.

In case the second data processing system generates the decompressed data, the CT reconstruction is for example generated by the second data processing system or by a third data processing system, which receives the decompressed data from the second data processing system. In case the first data processing system generates the decompressed data, the CT reconstruction is for example generated by the first data processing system or by the third data processing system, which receives the decompressed data from the first data processing system.

In particular, a known method for CT reconstruction may be used to generate the CT reconstruction depending on the decompressed data.

Further implementations of the computer-implemented method for generating a CT reconstruction according to the invention follow directly from the various embodiments of the computer-implemented method for compression or for compression and decompression according to the invention and vice versa.

According to a further aspect of the invention, a data processing arrangement, which comprises a first data processing system is provided. The first data processing system is adapted to carry out a computer-implemented method for compressing data of a CT system according to the invention.

In the present disclosure, the expressions "data processing system" and "at least one data processing device" may be used interchangeably. A data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to several implementations of the data processing arrangement, the first data processing system is adapted to carry out a computer-implemented method for compressing and decompressing data of a CT system according to the invention, which includes, in particular, generating the decompressed data, as described.

According to several implementations of the data processing arrangement, it comprises a second data processing system. The first data processing system and the second data processing system are adapted to commonly carry out a computer-implemented method for compressing and decompressing data of a CT system according to the invention.

This can be understood as follows: The first data processing system is adapted to carry out the computer-implemented method for compressing the data of the CT system and to transmit the fully compressed data to the second data processing system or to store the fully compressed data to the storage device. The second data processing system is adapted to receive the fully compressed data from the first data processing system or to read it from the storage device. The second data processing system is further adapted to carry out the remaining steps of the computer-implemented method for compressing and decompressing data of a CT system, in particular to reverse the lossless compression method and to reverse the lossy compression method.

According to a further aspect of the invention, a CT system comprising a CT scanner and a data processing arrangement according to the invention is provided.

The CT scanner comprises the X-ray detector, which is configured to generate the detector data. For example, the CT scanner may comprise the first data processing system and the transmission link, in particular the slip ring. The slip ring can be a contact-based slip ring or a contactless slip ring.

In implementations of the CT system where the data processing arrangement also comprises the second data processing system, the second data processing system may be comprised by the CT scanner as well or may be provided separately from the CT scanner. It is also possible that a part of the second data processing system is comprised by the CT scanner and another part of the second data processing system may be provided separately from the CT scanner.

According to several implementations, the X-ray detector is an integrating X-ray detector. In other words, the X-ray detector is not a photon-counting X-ray detector.

For example, for each detector pixel of the plurality of detector pixels, the respective pixel value corresponds to a measure of the intensity of X-rays incident on the respective detector pixel integrated over a data acquisition period, also referred to as a frame or detector frame.

According to at least one embodiment, the X-ray detector is a photon-counting X-ray detector, also denoted as spectral X-ray detector.

For example, for each detector pixel of the plurality of detector pixels, the respective pixel value corresponds to a number of detections of X-ray photons with an energy of at least a predefined energy threshold value incident on the respective detector pixel during a data acquisition period, also referred to as a frame or detector frame. In particular, more than one pixel value may be generated for each detector pixel, the different pixel values corresponding to different energy threshold values.

According to a further aspect of the invention, a first computer program comprising first instructions is provided. When the first instructions are executed by a first data processing system, the first instructions cause the first data processing system to carry out a computer-implemented method for compressing data of a CT system according to the invention.

The first instructions may be provided as program code, for example. The program code can for example be provided as binary code or assembler and/or as source code of a programming language, for example C, and/or as program script, for example Python.

According to a further aspect of the invention, a second computer program comprising second instructions is provided. When the second instructions are executed by a second data processing system, the second instructions cause the second data processing system to receive fully compressed data from a first data processing system or to read out the fully compressed data from the storage device. The second instructions further cause the second data processing system to recover the pre-compressed data based on the fully compressed data by reversing the lossless compression method and to generate decompressed data by the second data processing system based on the recovered pre-compressed data by reversing the lossy compression method.

In other words, when the first instructions are executed by the first data processing system and the second instructions are executed by the second data processing system, the first and the second instructions cause the first data processing system and the second data processing system to commonly carry out a computer-implemented method for compressing and decompressing data of a CT system according to the invention.

The second instructions may be provided as program code, for example. The program code can for example be provided as binary code or assembler and/or as source code of a programming language, for example C, and/or as program script, for example Python.

According to a further aspect of the invention, a first computer-readable storage medium storing a first computer program according to the invention is provided.

According to a further aspect of the invention, a second computer-readable storage medium storing a second computer program according to the invention is provided.

According to a further aspect of the invention, a computer program product comprising or consisting of a first computer-readable storage medium according to the invention and a second computer-readable storage medium according to the invention is provided.

The first computer program, the second computer program, the first computer-readable storage medium, and the second computer-readable storage are respective computer program products comprising the first instructions or the second instructions, respectively.

Above and in the following, the solution according to the invention is described with respect to the claimed systems as well as with respect to the claimed methods. Features, advantages, or alternative embodiments herein can be assigned to the other claimed objects and vice versa. In other words, claims and embodiments for the systems can be improved with features described or claimed in the context of the respective methods. In this case, the functional features of the method are implemented by physical units of the system.

Further features and feature combinations of the invention are obtained from the figures and their description as well as from the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,

FIG 1   shows a schematic representation of an exemplary implementation of a CT system according to the invention;

FIG 2   shows a flow diagram of an exemplary implementation of a computer-implemented method for compressing data of a CT system according to the invention;

FIG 3   shows a flow diagram of an exemplary implementation of a computer-implemented method for generating a CT reconstruction according to the invention;

FIG 4   shows a flow diagram of a further exemplary implementation of a computer-implemented method for compressing data of a CT system according to the invention;

FIG 5   shows a flow diagram of a part of an exemplary implementation of a computer-implemented method for compressing and decompressing data of a CT system according to the invention; and

FIG 6   shows schematically an artificial neural network.

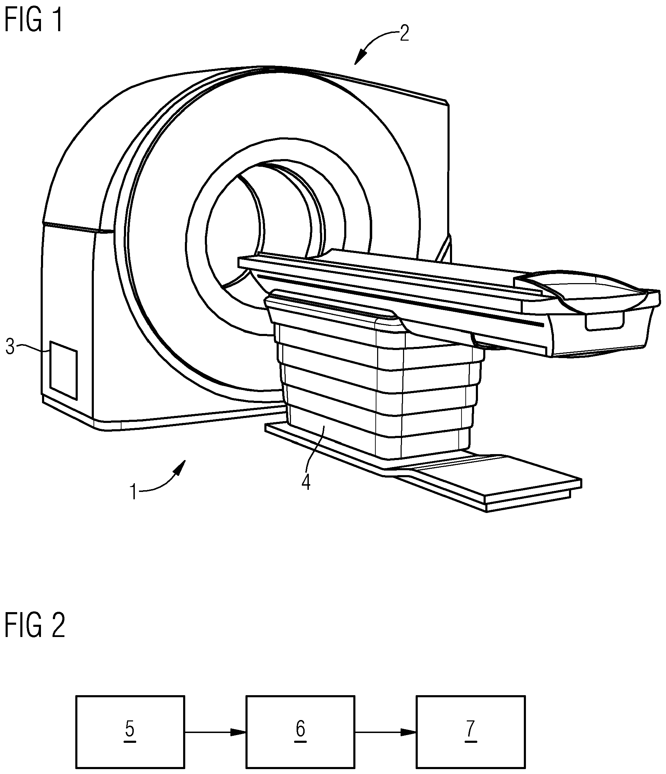

FIG 1 shows a schematic representation of an exemplary implementation of a CT system 1 according to the invention.

The CT system 1 comprises a CT scanner 2, also referred to as a gantry, which comprises an X-ray source and an X-ray detector, wherein the X-ray detector contains a detector pixel array with a plurality of detector pixels. The CT system 1 comprises a data processing arrangement 3 according to the invention. Therein, a first data processing system 12 of the data processing arrangement 3 is comprised by the CT scanner 2 and, in particular, arranged in a rotating part of the CT scanner 2. A second data processing system 13 of the data processing arrangement 3 is arranged separately from the first data processing system 12. The second data processing system 13 may be comprised or partially comprised by a stationary part of the CT scanner 2. The second data processing system 13 may also be provided separately from the CT scanner 2 or in part separately from the CT scanner 2. The first data processing system 12 and the second data processing system 13 may be connected to each other for data transfer via a transmission link 14, in particular a slip ring of the CT scanner 2.

By means of the CT scanner 2, X-ray projections can be recorded from several directions. Based on the different attenuation of the X-rays by different areas of the object to be imaged, which is introduced into the recording area of the CT scanner 2, for example via a patient table 4, the material composition of the scanned region can be inferred using computer-based reconstruction methods. The detector data, which in particular represent the X-ray projections, are generated by the CT scanner 2 and transmitted via the transmission link 14 from the first data processing system 12 to the second data processing system 13.

The first data processing system 12 and the second data processing system 13 are adapted to commonly carry out a computer-implemented method for compressing and decompressing data of the CT system 1 according to the invention. Therein, the first data processing system 12 is adapted to carry out a computer-implemented method for compressing data of the CT system 1 according to the invention. In particular, the first data processing system 12 may comprise one or more ASICs and/or one or more FPGAs, which are adapted to carry out the computer-implemented method for compressing data of the CT system 1 according to the invention. The first data processing system 12 may, alternatively or in addition, comprise one or more CPUs, which are adapted to carry out the computer-implemented method for compressing data of the CT system 1 according to the invention.

A flow diagram of a computer-implemented method for compressing data of the CT system 1 according to the invention is shown in FIG 2. Therein, detector data 5 is received from the X-ray detector of the CT system 1. Pre-compressed data 6 is generated by compressing the detector data 5 using a lossy compression method. Fully compressed data 7 is generated depending on the pre-compressed data 6 using a lossless compression method, for example an entropy coding method such as an ANS method.

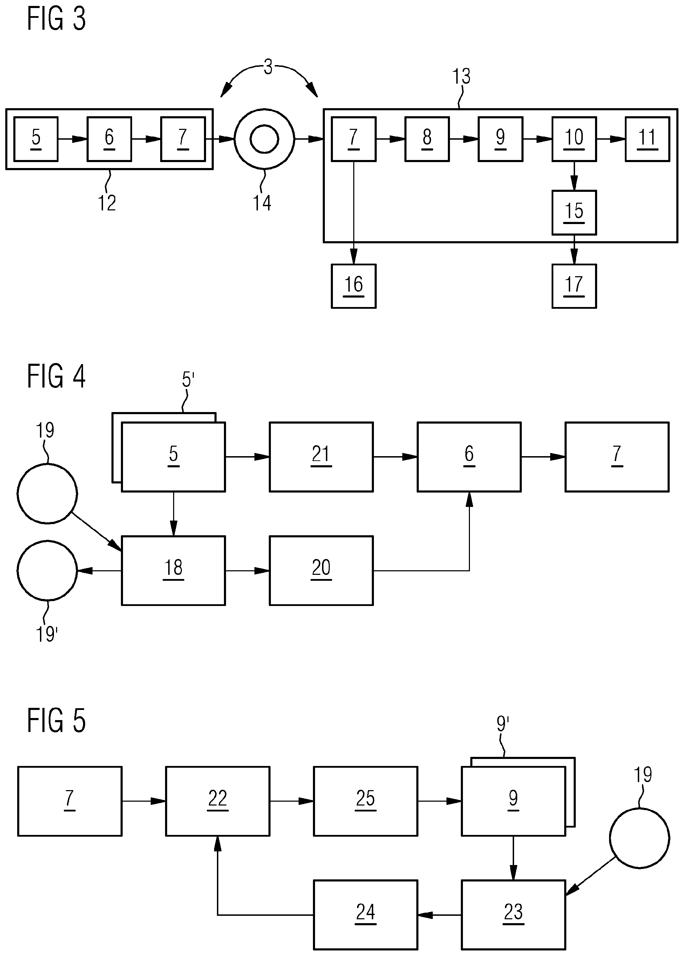

FIG 3 shows a flow diagram of a computer-implemented method for generating a CT reconstruction 11, wherein a computer-implemented method as described with respect to FIG 2 is carried out by the first data processing system 12 and the fully compressed data 7 is transmitted from the first data processing system 12 to the second data processing system 13 via the transmission link 14. The detector data 5 as well as the fully compressed data 7 are, in particular, integer data, for example multi-dimensional integer data. The pre-compressed data 6 is recovered by the second data processing system 13 based on the fully compressed data 7 by reversing the lossless compression method and decompressed data 9 is generated by the second data processing system 13 based on the recovered pre-compressed data 8 by reversing the lossy compression method. The CT reconstruction 11 is generated by the second data processing system 13 depending on the decompressed data.

For example, the second data processing system 13 may also store the fully compressed data 7 to a storage device 16, in particular for short-term storage. In some implementations, the second data processing system 13 may generate pre-processed data 10 based on the decompressed data 9 and generate the CT reconstruction 11 based on the pre-processed data 10. The pre-processed data 10 may be float data, in particular multi-dimensional float data.

Optionally, the second data processing system 13 may store the pre-processed data 10 to a further storage device 17, for example for long-term storage. Alternatively or in addition, the second data processing system 13 may generate sampled data 15 by sampling the pre-processed data 10, for example linearly, and store the sampled data 15 to the further storage device 17, for example for long-term storage. The sampled data 15 is integer data, in particular multi-dimensional integer data.

A flow diagram of a further computer-implemented method for compressing data of the CT system 1 according to the invention, which may for example be used in the computer-implemented method of FIG 3, is shown in FIG 4.

Therein, the detector data 5 comprises a respective pixel value x for each of a plurality of detector pixels of the X-ray detector and the lossy compression method comprises a pixel-wise lossy compression, wherein a respective pre-compressed pixel value $L(x)$ is computed for each detector pixel c of the plurality of detector pixels using a predefined compression function L. FIG 4 depicts the detector data 5 of a current frame, which is to be compressed, as well as further detector data 5' of a previous frame, which has already been compressed using the same method.

For example, for each detector pixel c of the plurality of detector pixels, historic pixel values $h_c$ of each environment pixel within a predefined historic environment $H_c$ of the detector pixel c may be received. A predictor value $P_c$ for the detector pixel c is computed depending on the historic pixel values $h_c$ of the environment pixels of the detector pixel c and independent of the pixel value x itself. The pre-compressed data 6 is generated depending on the pre-compressed pixel value $L(x)$ and the predictor value $P_c$.

The historic environment $H_c$ for a detector pixel c is, in particular, a set of N uncompressed pixel values $x_i$ of detector pixels that are already compressed at the time when detector pixel c is about to be compressed. This assures that during decompression, these values are available when detector pixel c is about to be decompressed. In general, these may be pixels close to the current pixel c with a strong correlation with the current pixel value x. However, this is not necessarily the case. It is, in particular, also possible that the historic environment $H_c$ includes detector pixels from other frames, for example from the further detector data 5'. When starting with the compression of the initial detector pixel, the historic environment $H_c$ may be empty. It is noted that the historic environment $H_c$ does not include the current pixel c itself.

The compression function L maps the pixel value x to a pre-compressed pixel-value y, which is an integer in the interval $[0, 2^m - 1]$, wherein m is a predefined bit depth. The compression function L fulfils, in particular, $L(L^{-1}(L(x))) = L(x)$ for any pixel value x. In other words, the inverse compression function $L^{-1}$ is bijective on the domain of integers within $[0, 2^m - 1]$. For example, the compression function L may be a square root function or may be partly a square root function and partly a linear function. It is also possible, in some implementations, that the compression function L maps an argument, which contains the pixel value x as well as the historic pixel values $h_c$, to the pre-compressed pixel-value y.

The predictor value $P_c$ can be considered as an estimate for the current pixel to be compressed. The predictor value $P_c$ may be a weighted sum or, for example, may be calculated using a trained MLM, such as a trained ANN, in particular a deep ANN. The predictor value $P_c$ is, in particular, computed depending on a set of predefined parameters 19, which may be the respective coefficients of the weighted sum or comprise the respective network parameters, in particular weights and biases, of the ANN.

In some implementations, the parameters 19 may be updated yielding updated parameters 19' according to a predefined update rule. The update may be carried out for each pixel or each predefined group of pixels, et cetera.

In case the parameters 19 are coefficients for the weighted sum, the update may be carried out, for example, by minimizing a cost function, which depends for example on the quadratic error between the pixel value x and the corresponding predictor value, summed over a plurality of pixels. In case the parameters 19 are network parameters of the ANN, the update may be carried out by further training the ANN depending on the predictor value using the pixel value x as ground truth label.

As a result of the pixel-wise application of the compression function L to the detector data, pre-compressed pixel values 21 are obtained and as a result of the pixel-wise computation of the predictor values, predictors 18 are obtained. By applying the compression function L to the predictors 18, pre-compressed predictors 20 are generated. The pre-compressed data 6 may then be generated by computing a respective difference value between each pre-compressed pixel value L(x) and the corresponding pre-compressed predictor value $L(P_c)$ for the respective detector pixel c. The offset difference value may be given by

$$(L(x) - L(P_c) + 2^m) \bmod 2^m.$$

This results, in particular, in many different values close to zero or close to $2^m-1$. Thus, the pre-compressed data 6 has a low entropy and can therefore be compressed particularly well by entropy coding methods. The fully compressed data 7 is then computed by applying the lossless compression method, for example ANS, to the pre-compressed data 6. This may be done frame by frame or batch-wise for one batch of frames after the other. The batch of frames may comprise tens or hundreds of frames, for example 64 frames, 128 frames or 256 frames. It is also possible that only one sub-frame is compressed, or that batches of sub-frames are compressed, for example under the assumption that outer detector areas behave differently than inner ones.

A flow diagram of the decompression part of a computer-implemented method for compressing and decompressing data of the CT system 1 according to the invention, which may for example be used in the computer-implemented method of FIG 3, is shown in FIG 5. In particular, the fully compressed data 7 may have been generated as described with respect to FIG 4.

The decompression process can be widely considered as an inversion of the compression process. The inverse lossless compression, in particular inverse ANS, is used to generate the recovered pre-compressed data 8. The lossy compression method is reversed by using a pixel-wise lossy decompression, wherein a respective decompressed pixel value is computed for each detector pixel of the plurality of detector pixels using $L^{-1}$. The predictor values are re-computed for each detector pixel depending on the historic pixel values of the environment pixels of the respective detector pixel. The respective decompressed pixel values are computed depending on the recovered pre-compressed data 8 and the re-computed predictor values 23. In particular, the pre-compressed re-computed predictor values 24 are pre-compressed using the compression function L.

In particular, respective sum value *mod* $2^m$ of the recovered difference value for the respective detector pixel and the pre-compressed re-computed predictor value 24 is computed in a processing step 22. As a result of carrying out this process for all detector pixels and the current frame, prepared data 25 is generated. The decompressed data 9 is then computed by applying the inverse compression function $L^{-1}$ to the sum values of the prepared data 25. FIG 5 shows also further decompressed data 9' of the previous frame. It is noted that the reversed lossy compression is, for example, carried out frame-by-frame and pixel-by-pixel in the same order as the lossy compression has been carried out. Thus, the predictor values can always be re-computed as during the lossy compression.

In several embodiments of the invention, an improved compression ratio is achieved. A prototype to compress raw data of a photon-counting CT scanner could increase the compression ratio compared to existing lossy methods by roughly 60% without further data degradation. In particular, the data loss due to the lossy part of the method was not increased compared to the existing lossy method or, in other words, the increased compression ratio is not achieved at the cost of increased data degradation.

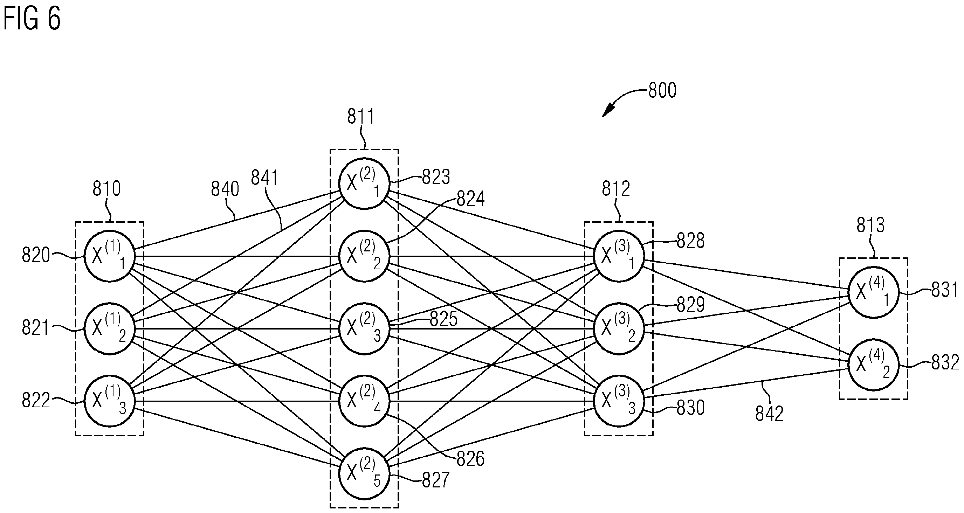

FIG 6 displays an embodiment of an ANN 800, which is for example designed as an MLP. The ANN 800 comprises nodes 820, ..., 832 and edges 840, ..., 842, wherein each edge 840, ..., 842 is a directed connection from a first node 820, ..., 832 to a second node 820, ..., 832. In general, the first node 820, ..., 832 and the second node 820, ..., 832 are different nodes 820, ..., 832. It is, however, also possible that the first node 820, ..., 832 and the second node 820, ..., 832 are identical. For example, in FIG 6, the edge 840 is a directed connection from the node 820 to the node 823, and the edge 842 is a directed connection from the node 830 to the node 832. An edge 840, ..., 842 from a first node 820, ..., 832 to a second node 820, ..., 832 is also denoted as ingoing edge for the second node 820, ..., 832 and as outgoing edge for the first node 820, ..., 832.

In this example, the nodes 820, ..., 832 of the artificial neural network 800 can be arranged in layers 810, ..., 813, wherein the layers can comprise an intrinsic order introduced by the edges 840, ..., 842 between the nodes 820, ..., 832. In particular, edges 840, ..., 842 can exist only between neighboring layers of nodes. In the displayed example, there is an input layer 810 comprising only nodes 820, ..., 822 without an incoming edge, an output layer 813 comprising only nodes 831, 832 without outgoing edges, and hidden layers 811, 812 in-between the input layer 810 and the output layer 813. In general, the number of hidden layers 811, 812 can be chosen arbitrarily. In an MLP, this number is at least one. The number of nodes 820, ..., 822 within the input layer 810 usually relates to the number of input values of the artificial neural network 800, and the number of nodes 831, 832 within the output layer 813 usually relates to the number of output values of the artificial neural network 800.

In particular, a real number can be assigned as a value to every node 820, ..., 832 of the artificial neural network 800. Here, $x^{(n)}_i$ denotes the value of the i-th node 820, ..., 832 of the n-th layer 810, ... , 813. The values of the nodes 820, ... , 822 of the input layer 810 are equivalent to the input values of the artificial neural network 800. The values of the nodes 831, 832 of the output layer 813 are equivalent to the output value of the artificial neural network 800. Furthermore, each edge 840, ... , 842 can comprise a weight being a real number. In particular, the weight is a real number within the interval [-1, 1] or within the interval [0, 1]. Here, $w^{(m,n)}_{i,j}$ denotes the weight of the edge between the i-th node 820, ..., 832 of the m-th layer 810, ..., 813 and the j-th node 820, ..., 832 of the n-th layer 810, ..., 813. Furthermore, the abbreviation $w^{(n)}_{i,j}$ is defined for the weight $w^{(n,n+1)}_{i,j}$. In particular, to calculate the output values of the neural network 800, the input values are propagated through the neural network 800. In particular, the values of the nodes 820, ... , 832 of the (n+1)-th layer 810, ..., 813 can be calculated based on the values of the nodes 820, ... , 832 of the n-th layer 810, 813 by

$$x^{(n+1)}_j = f\left(\sum_i x^{(n)}_i \; w^{(n)}_{i,j}\right).$$

Herein, the function f is denoted as transfer function or activation function. Known transfer functions are step functions, the sigmoid functions, for example the logistic function, the generalized logistic function, the hyperbolic tangent, the arctangent function, the error function, the smoothstep function, or rectifier functions. The transfer function is for example used for normalization purposes. In particular, the values are propagated layer-wise through the neural network 800, wherein values of the input layer 810 are given by the input of the neural network 800, wherein values of the first hidden layer 811 can be calculated based on the values of the input layer 810 of the neural network 800, wherein values of the second hidden layer 812 can be calculated based on the values of the first hidden layer 811, and so forth.

In order to set the values $w^{(m,n)}_{i,j}$ for the edges, the neural network 800 has to be trained using training data. In particular, training data comprises training input data and training output data (denoted as $t_i$). For a training step, the neural network 800 is applied to the training input data to generate calculated output data. In particular, the training data and the calculated output data comprise a number of values, said number being equal with the number of nodes of the output layer. In particular, a comparison between the calculated output data and the training data is used to recursively adapt the weights within the neural network 800 (backpropagation algorithm). In particular, the weights are changed according to

$$w'^{(n)}_{i,j} = w^{(n)}_{i,j} - \gamma \, \delta^{(n)}_j \, x^{(n)}_i,$$

wherein $\gamma$ is a predefined learning rate, and the numbers $\delta^{(n)}_j$ can be recursively calculated as

$$\delta^{(n)}_j = \left(\sum_k \delta^{(n+1)}_k \, w^{(n+1)}_{j,k}\right) f'\left(x^{(n)}_i w^{(n)}_{i,j}\right)$$

based on $\delta^{(n+1)}_j$, if the (n+1)-th layer is not the output layer 813, and

$$\delta^{(n)}_j = \left(x^{(n+1)}_j - t^{(n+1)}_j\right) \, f'\left(x^{(n)}_i w^{(n)}_{i,j}\right),$$

if the (n+1)-th layer is the output layer 813, wherein f' is the first derivative of the activation function, and $t^{(n+1)}_j$ is the comparison training value for the j-th node of the output layer 813.

Independent of the grammatical term usage, individuals with male, female or other gender identities are included within the term.

**Claims**

1. Computer-implemented method for compressing data of a computed tomography, CT, system (1), wherein

   - detector data (5) is received from an X-ray detector of the CT system (1);
   - pre-compressed data (6) is generated by compressing the detector data (5) using a lossy compression method; and
   - fully compressed data (7) is generated depending on the pre-compressed data (6) using a lossless compression method.

2. Computer-implemented method according to claim 1, wherein

   - the detector data (5) corresponds to a frame of a frame sequence, which comprises the frame and at least one further frame;
   - for each of the at least one further frame, respective further detector data (5') is received and respective further pre-compressed data (6) is generated by compressing the respective further detector data (5') using the lossy compression method; and
- the fully compressed data (7) is generated by compressing joint pre-compressed data (6), which comprises the pre-compressed data (6) for the frame and the further pre-compressed data for the at least one further frame.

3. Computer-implemented method according to one of the preceding claims, wherein

- the lossless compression method is an entropy coding method; and/or
- the lossless compression method is an asymmetric numeral systems, ANS, method.

4. Computer-implemented method according to one of the preceding claims, wherein the detector data (5) comprises a respective pixel value for each of a plurality of detector pixels of the X-ray detector and the lossy compression method comprises a pixel-wise lossy compression, wherein a respective pre-compressed pixel value is computed for each detector pixel of the plurality of detector pixels.

5. Computer-implemented method according to claim 4, wherein the pixel-wise lossy compression comprises, for each detector pixel of the plurality of detector pixels, computing the pre-compressed pixel value for the respective detector pixel using a predefined compression function, whose argument depends on the respective pixel value.

6. Computer-implemented method according to one of claims 4 or 5, wherein the pixel-wise lossy compression comprises, for each detector pixel of the plurality of detector pixels,

- receiving historic pixel values of each environment pixel within a predefined historic environment of the respective detector pixel;
- computing a predictor value (18) for the respective detector pixel depending on the historic pixel values of the environment pixels of the respective detector pixel and independent of the pixel value of the respective detector pixel; and
- generating the pre-compressed data (6) depending on the pre-compressed pixel value for the respective detector pixel and the predictor value (18) for the respective detector pixel.

7. Computer-implemented method according to claim 6, wherein, for each detector pixel of the plurality of detector pixels,

- a pre-compressed predictor value (20) is computed based on the predictor value (18) for the respective detector pixel;

- a difference value between the pre-compressed pixel value for the respective detector pixel and the pre-compressed predictor value (20) for the respective detector pixel is computed; and
- the pre-compressed data (6) is generated depending on the difference values for the plurality of detector pixels.

8. Computer-implemented method according to one of claims 6 or 7, wherein

- the predictor value (18) for the respective detector pixel is computed as a weighted sum of the historic pixel values; or
- the predictor value (18) for the respective detector pixel by applying a trained machine learning model to input data comprising the historic pixel values.

9. Computer-implemented method for compressing and decompressing data of a CT system (1), wherein a computer-implemented method for compressing the data of the CT system (1) according to one of the preceding claims is carried out by a first data processing system (12), and

- the fully compressed data (7) is transmitted from the first data processing system (12) to a second data processing system (13), the pre-compressed data (6) is recovered by the second data processing system (13) based on the fully compressed data (7) by reversing the lossless compression method, and decompressed data (9) is generated by the second data processing system (13) based on the recovered pre-compressed data (8) by reversing the lossy compression method; or
- the fully compressed data (7) is stored to a storage device by the first data processing system (12) and the fully compressed data (7) is read out from the storage device by a second data processing system (13), the pre-compressed data (6) is recovered by the second data processing system (13) based on the fully compressed data (7) by reversing the lossless compression method, and decompressed data (9) is generated by the second data processing system (13) based on the recovered pre-compressed data (8) by reversing the lossy compression method; or
- the fully compressed data (7) is stored to a storage device by the first data processing system (12) and the fully compressed data (7) is read out from the storage device by the first data processing system (13), the pre-compressed data (6) is recovered by the first data processing system (13) based on the fully compressed data

(7) by reversing the lossless compression method, and decompressed data (9) is generated by the first data processing system (13) based on the recovered pre-compressed data (8) by reversing the lossy compression method.

10. Computer-implemented method according to claim 9, wherein

- the computer-implemented method for compressing the data of the CT system (1) is carried out according to one of claims 6 to 8;
- reversing the lossy compression method comprises a pixel-wise lossy decompression, wherein a respective decompressed pixel value is computed for each detector pixel of the plurality of detector pixels;
- the pixel-wise lossy decompression comprises, for each detector pixel of the plurality of detector pixels, receiving recovered historic pixel values of each environment pixel within the historic environment of the respective detector pixel, re-computing the predictor value (23) for the respective detector pixel depending on the recovered historic pixel values of the environment pixels of the respective detector pixel, and computing the respective decompressed pixel value depending on the recovered pre-compressed data (8) and the recomputed predictor value (23) for the respective detector pixel.

11. Computer-implemented method according to claim 10, wherein

- the computer-implemented method for compressing the data of the CT system (1) is carried out according to claim 7;
- a further pre-compressed predictor value (24) is computed based on the re-computed predictor value (23);
- for each detector pixel of the plurality of detector pixels, the difference value for the respective detector pixel is recovered based on the recovered pre-compressed data (8), a sum value of the recovered difference value for the respective detector pixel and the further pre-compressed predictor value (24) for the respective detector pixel is computed, and the respective decompressed pixel value is computed depending on the sum value.

12. Computer-implemented method according to claim 11, wherein

- the computer-implemented method for compressing the data of the CT system (1) is carried out according to claim 5; and
- the respective decompressed pixel value is computed applying an inverse of the compression function to an argument, which depends on the respective sum value.

13. Computer-implemented method for generating a CT reconstruction (11), wherein

- a computer-implemented method for compressing and decompressing data of a CT system (1) according to one of claims 9 to 12 is carried out; and
- the CT reconstruction (11) is generated depending on the decompressed data.

14. Data processing arrangement (3), which comprises

- a first data processing system (12) adapted to carry out a computer-implemented method according to one of claims 1 to 13; or
- a first data processing system (12) and a second data processing system (13) adapted to commonly carry out a computer-implemented method according to one of claims 9 to 13.

15. CT system (1) comprising

- a CT scanner (2), which comprises an X-ray detector, which is configured to generate detector data (5), and a first data processing system (12) adapted to carry out a computer-implemented method according to one of claims 1 to 13; or
- a CT scanner (2), which comprises an X-ray detector, which is configured to generate detector data (5), and a first data processing system (12), the CT system (1) further comprising a second data processing system (13), wherein the first data processing system (12) and the second data processing system (13) are adapted to commonly carry out a computer-implemented method according to one of claims 9 to 13.

16. Computer program product comprising

- first instructions, which, when carried out by a first data processing system (12), cause the first data processing system (12) to carry out a computer-implemented method according to one of claims 1 to 13; or
- first instructions and second instruction, which, when the first instructions are carried out by a first data processing system (12) and the second instructions are carried out by a first data processing system (12), cause the first data processing system (12) and the first data processing system (12) to commonly carry out a method according to one of claims 9 to 13.

FIG 1

FIG 2

FIG 3

FIG 4

FIG 5

FIG 6

Europäisches
Patentamt

European
Patent Office

Office européen
des brevets

# EUROPEAN SEARCH REPORT

**Application Number**

EP 24 16 8719

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JEBARAJ SAM DEVAVARAM ET AL: "JPEG-XL based Compression of DICOM Images for Reduced Storage and Transmission Costs", 2023 3RD INTERNATIONAL CONFERENCE ON INTELLIGENT TECHNOLOGIES (CONIT), IEEE, 23 June 2023 (2023-06-23), pages 1-6, XP034394810, DOI: 10.1109/CONIT59222.2023.10205928 [retrieved on 2023-08-07] | 1,3,9, 13-16 | INV. H04N19/593 H04N19/91 G03B42/02 A61B6/00 |
| Y | * abstract; figure 1 * | 2 | |
| A | * Sections I-III, V * | 4-8, 10-12 | |
| A | ALAKUIJALA JYRKI ET AL: "JPEG XL next-generation image compression architecture and coding tools", APPLICATIONS OF DIGITAL IMAGE PROCESSING XLII, 6 September 2019 (2019-09-06), XP093202044, DOI: 10.1117/12.2529237 * abstract; figure 4 * * Sections 1 and 4 * | 1-16 | |
| Y | US 10 687 062 B1 (RAMASWAMY SHARADH [US]) 16 June 2020 (2020-06-16) | 2 | |
| A | * column 1; figure 4 * * columns 11, 13 * | 1,14,16 | |
| Y,D | US 9 078 625 B2 (KAPPLER STEFFEN [DE]; KRAFT EDGAR [DE] ET AL.) 14 July 2015 (2015-07-14) | 5,7,11, 12 | |
| A | * abstract; figures 1-4 * * column 6 - column 8 * | 1-4, 8-10, 13-16 | |

**TECHNICAL FIELDS SEARCHED (IPC)**

H04N
G03D
G03B
A61B

-/--

The present search report has been drawn up for all claims

| Place of search | Date of completion of the search | Examiner |
|---|---|---|
| The Hague | 30 September 2024 | Streich, Sebastian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2011/170789 A1 (AMON PETER [DE] ET AL) 14 July 2011 (2011-07-14)<br>* abstract; figures 1, 3 *<br>* paragraph [0004] *<br>* paragraph [0032] - paragraph [0033] *<br>* paragraph [0041] - paragraph [0050] *<br>----- | 1,4,6,8, 10<br>5,7,11, 12 | |
| A | BARZEN BENJAMIN LUKAS CAJUS ET AL: "Accelerated Deep Lossless Image Coding with Unified Paralleleized GPU Coding Architecture",<br>2022 PICTURE CODING SYMPOSIUM (PCS), IEEE, 7 December 2022 (2022-12-07), pages 109-113, XP034279205,<br>DOI: 10.1109/PCS56426.2022.10018050<br>[retrieved on 2023-01-20]<br>* abstract; figure 2 *<br>----- | 8 | |

| | TECHNICAL FIELDS SEARCHED (IPC) |
|---|---|

The present search report has been drawn up for all claims

| Place of search | Date of completion of the search | Examiner |
|---|---|---|
| The Hague | 30 September 2024 | Streich, Sebastian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

EP 24 16 8719

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

**Application Number**

EP 24 16 8719

The Search Division considers that the present European patent application does not comply with the requirements of unity of invention and relates to several inventions or groups of inventions, namely:

```
1. claims: 1-3(completely); 9, 13-16(partially)

      joint lossless compression across frames
                    - - -

2. claims: 4-8, 10-12(completely); 9, 13-16(partially)

      pixel-wise lossy compression
                    - - -
```

**ANNEX TO THE EUROPEAN SEARCH REPORT
ON EUROPEAN PATENT APPLICATION NO.**          EP 24 16 8719

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2024

| Patent document cited in search report | | Publication date | Patent family member(s) | | Publication date |
|---|---|---|---|---|---|
| US 10687062 | B1 | 16-06-2020 | CN | 113454995 A | 28-09-2021 |
| | | | EP | 3928515 A1 | 29-12-2021 |
| | | | JP | 7147075 B2 | 04-10-2022 |
| | | | JP | 2022510733 A | 27-01-2022 |
| | | | KR | 20210113681 A | 16-09-2021 |
| | | | US | 10687062 B1 | 16-06-2020 |
| | | | WO | 2020171860 A1 | 27-08-2020 |
| US 9078625 | B2 | 14-07-2015 | CN | 102525532 A | 04-07-2012 |
| | | | DE | 102010063435 A1 | 21-06-2012 |
| | | | US | 2012158811 A1 | 21-06-2012 |
| US 2011170789 | A1 | 14-07-2011 | CN | 102124495 A | 13-07-2011 |
| | | | DE | 102008058489 A1 | 15-04-2010 |
| | | | EP | 2313864 A1 | 27-04-2011 |
| | | | JP | 5284471 B2 | 11-09-2013 |
| | | | JP | 2012500546 A | 05-01-2012 |
| | | | KR | 20110063768 A | 14-06-2011 |
| | | | US | 2011170789 A1 | 14-07-2011 |
| | | | WO | 2010020592 A1 | 25-02-2010 |

EPO FORM P0459

For more details about this annex : see Official Journal of the European Patent Office, No. 12/82

**REFERENCES CITED IN THE DESCRIPTION**

**Patent documents cited in the description**

- US 9078625 B2